# EUROPEAN PATENT APPLICATION

(11) **EP 3 991 801 A1**
(43) Date of publication of application: **04.05.2022**
(21) Application number: 20205504.2
(22) Date of filing: 03.11.2020
(51) Int. Cl.: A61Q 19/08, A61K 8/9789

(54) **COMPOSITION FOR IMPROVING SKIN CONDITION CONTAINING PLANT EXTRACTS**

(71) Applicant: UAB "Bioklinika", 51364 Kaunas (LT)
(72) Inventor: Barasneviciute , Karolina, 51364 Kaunas (LT)
(74) Representative: Zaboliene, Reda

(57) **Abstract**

REFERENCES CITED

1. Dastmalchi K., Dorman H.J.D., Kosar M., Hiltunen R. (2007) Chemical composition and *in vitro* antioxidant evaluation of a water-soluble Moldavian balm (*Dracocephalum moldavica L.*) extract. LWT Food Sci Technol 40:239-248.
2. Povilaityte' V, Cuvelier ME, Berset C (2001) Antioxidant properties of Moldavian dragonhead (*Dracocephalum moldavica L.*)*.* J Food Lipids 8:45-64.
3. Higuera-Ciapara, I.; Félix-Valenzuela, L.; Goycoolea, F.M. Astaxanthin: A review of its chemistry and applications. Crit. Rev. Food Sci. Nutr. 2006, 46, 185-196.
4. Davinelli, S., Nielsen, M., & Scapagnini, G. (2018). Astaxanthin in skin health, repair, & disease: a comprehensive review. Nutrients., 10, 521-533.
5. Silva, T.H.; Alves, A.; Ferreira, B.; Oliveira, J.M.; Reys, L.; Ferreira, R.; Sousa, R.; Silva, S.; Mano, J.; Reis, R. Materials of marine origin: A review on polymers and ceramics of biomedical interest. Int. Mater. Rev. 2012, 57, 276-306.
6. De Luca C., Mikhal'chik E.V., Suprun M.V., *et al.* Skin antiaging and systemic redox effects of supplementation with marine collagen peptides and plant-derived antioxidants: a single-blind case-control clinical study. Oxid Med Cell Longev. 2016; 2016:4389410.
7. Han X., Parker T.L. Antiinflammatory Activity of Cinnamon (*Cinnamomum zeylanicum*) Bark Essential Oil in a Human Skin Disease Mode. Phytother. Res. 2017; 31(7):1034-1038.
8. Takasao N., Tsuji-Naito K., Ishikura S., *et al.* Cinnamon extract promotes type I collagen biosynthesis via activation of IGF-I signaling in human dermal fibroblasts. J Agric Food Chem 2012; 60(5):1193-200.
9. Borkow G. Using Copper to Improve the Well-Being of the Skin. Current Chemical Biology, 2014, 8, 89-102.
10. Lin C.C., Lu J.M., Yang J.J. et *al*. Anti-inflammatory and radical scavenge effects of *Arctium lappa.* Am. J. Chin. Med. 1996; 24:127-37.
11. Holetz F.B., Pessini G.L., Sanches N.R., *et al.* Screening of some plants used in the Brazilian folk medicine for the treatment of infectious diseases. Mem Inst Oswaldo Cruz 2002; 97:1027-31.
12. Food and Nutrition Board, Institute of Medicine. Dietary Reference Intakes for Vitamin C, Vitamin E, Selenium, and Carotenoids. Washington, DC: National Academy Press, 2000. Available at: http://www.nap.edu/books/0309069351/html/.
13. Dreno B., Trossaert M., Boiteau H.L., Litoux P. Zinc salts effects on granulocyte zinc concentration and chemotaxis in acne patients. Acta Derm Venereol 1992; 72:250-2.
14. Michaelsson G., Ljunghall K. Patients with dermatitis herpetiformis, acne, psoriasis and Darier's disease have low epidermal zinc concentrations. Acta Derm Venereol 1990; 70:304-8.
15. Myllyharju J. (2005) Intracellular post-translational modifications of collagens. Top. Curr. Chem. 247: 115-247.
16. Prussick R., Ali M.A., Rosenthal D., Guyatt G. The protective effect of vitamin E on the hemolysis associated with dapsone treatment in patients with dermatitis herpetiformis. Arch Dermatol. 1992; 128:210-3.
17. Mukhopadhyay D., Das M.K., Dhar S., Mukho-padhyay M. Multiple carboxylase deficiency (late onset) due to deficiency of biotinidase. Indian J Dermatol 2014; 59: 502-504.
18. Aguirre-Cruz G., Leon-Lopez A., Cruz-Gomez V., Jiménez-Alvarado R., Aguirre-Alvarez G. Collagen Hydrolysates for Skin Protection: Oral Administration and Topical Formulation. Antioxidants (Basel). 2020; 9(2):181. Published 2020 Feb 22. doi:10.3390/antiox9020181.
19. Stephens T.J., Sigler M.L., Hino P.D., Moigne A.L., Dispensa L. A Randomized, Double-blind, Placebo-controlled Clinical Trial Evaluating an Oral Anti-aging Skin Care Supplement for Treating Photodamaged Skin. J Clin Aesthet Dermatol. 2016; 9(4):25-32.
20. Verma K.C., Saini A.S., Dhamija S.K. Oral zinc therapy in acne vulgaris: a double blind trial. Acta Derm. Venereol. 1980; 60(4): 337-340.
21. Michaëlsson G., Juhlin L., Ljunghall K. A double-blind study of the effect of zinc and oxytetracycline in acne vulgaris. Br. J. Dermatol. 1977; 97(5):561-566. doi:10.1111/j.1365-2133.1977.tb14136.
22. Tominaga K., Hongo N., Fujishita M., Takahashi Y., Adachi Y. Protective effects of astaxanthin on skin deterioration. J. Clin. Biochem. Nutr. 2017; 61(1):33-39. doi:10.3164/jcbn.17-35.

The present invention relates to the composition of food supplement comprising balanced combination of nutritional materials useful in maintaining normal skin condition and acting sinergistically that has the unique research-proven anti-aging effect with antioxidant effects. The composition comprises extract of Moldavian dragonhead (*Dracocephalum moldavica*)*,* astaxanthin, common grapevines (*Vitis vinifera*) seed extract, extract of roots of greater burdock (*Arctium lappa*)*,* biotin, cinnamon, vitamin C from acerola cherry (*Malphigia glabra*)*,* as well as other vitamins and minerals which being in complex complement each other. The ingredients of food supplement composition are in 100 % active materials of plant origin, wihout any artificial additives, binders or excipients. The composition of the food supplement is administered taking into account the changing needs of the age-related skin.

## Description

### FIELD OF INVENTION

The present invention relates to the composition of food supplement comprising sinergistically acting derivative with balanced combination of nutritional materials useful in maintaining normal skin condition and that is for use to meet the changing needs of the skin related with ageing.

### BACKGROUND ART

As we age, there is a gradual increase in connective tissue damage such as destruction, oxidation and glycolysis of collagene. Such changes in the molecular level affect tissue integrity and promotes constant decrease in collagene mass, which ultimately leads to the formation of wrinkles and decreased skin elasticity. Such changes of skin condition related to aging are inevitable, but they may be postponed by choosing a healthy lifestyle, proper skin care from the outside and internal supply of the necessary materials.

Food supplements are for a long time used to stop skin ageing, improve its condition. This is proved by research in a randomized, double-blind, placebo-controlled clinical trial. Following a one-month wasting period, subjects received two anti-ageing skin care formula tablets (total daily dose: marine complex 210 mg, vitamin C 54 mg, zinc 4 mg) or placebo daily for 16 weeks. Subjects were assessed at weeks 6, 12, and 16 on clinical trial (0-10 VAS (visual analog scale)), bioinstrumentation, digital photography, and self-assessments. Finally, a conclusion was reached that women with photo-damaged skin receiving anti-ageing skin care supplement showed significant improvements in the skin condition [19].

CN201610212672 relates to moisturizing agent and the process for preparing thereof and belonges to skin care products. The moisturizing agent comprises raw materials in parts by mass as follows: 10-15 parts of sorbitol, 5-12 parts of propylene glycol, 6-14 parts of polyethylene glycol 4000, 8-18 parts of lanolin, 4-10 parts of urea, 1-4 parts of DL-alanine, 0.3-0.8 parts of vitamin C, 0.2-0.5 parts of 4-aminobenzoate, 0.5-1 part of a chamomile extract, 0.1-0.3 parts of betaine, 0.1-0.2 parts of burdock root, 0.3-1 part of silicone, 0.1-0.3 parts of rosemary, 0.4-1 part of monoglyceride and 10-20 parts of water. The moisturizing agent has excellent moisturizing and anti-allergic functions as well as a good biocompatibility, high availability and long moisturizing time.

US19980016800 relates to the pharmaceutical composition for the treatment of acne having an acne reduction component in an amount sufficient to reduce the redness and blemishes associated with acne. The invention also relates to pharmaceutical compositions having, in addition to the acne reduction component, a skin cell conditioning component in an amount sufficient to properly regulate the keratin and sebum production of the skin cells, thereby inhibiting the appearance of acne. Preferably, the skin cell conditioning component is a chromium component. In another form, the composition further includes at least one of a vitamin C source, burdock root, yellow dock root, horsetail extract, a catechin-based composition, a vitamin B1 source, a vitamin B2 source, a vitamin B3 source, a vitamin B5 source, and a vitamin E source. In a more preferred form, the invention also includes at least one amino acid component, a magnesium component, a selenium component, and biotin. The invention also relates to methods for treating acne by administering, alone or in conjunction with another composition, the pharmaceutical compositions in an amount therapeutically effective in reducing the incidence of acne and methods for additionally inhibiting the appearance of acne by conditioning skin cells.

EP3398585 provides a use of one or more components selected from the group consisting of L-ascorbic acid, a L-ascorbic acid derivative, and a salt thereof as a effective ingredient(s) in an external dermal composition for anti-aging, which composition further comprises an aqueous medium as a base material. The L-ascorbic acid derivative may be a glycosyl-, acyl-, or phosphorylated-derivative of L-ascorbic acid. Further provided is a method for producing an external dermal composition for anti-aging and a skin care.

The documents cited describes agents for improving the condition of the skin and stopping aging, but they only partially perform the above functions. Therefore, there is a need to create a composition that inhibits ageing and improves the condition of the skin by various mechanisms of action, which provide the ingredients of composition.

### SUMMARY OF THE INVENTION

The present invention aims to provide the composition of food supplement comprising balanced combination of nutritional materials useful in maintaining normal skin condition and acting sinergistically which has an unique research-proven anti-ageing effect with antioxidant effects. The composition is for oral use and comprises the following major components: extract of Moldavian dragonhead (*Dracocephalum moldavica*) (1:18), extract of roots of greater burdock (*Arctium lappa)* (4:1), acerola cherry (*Malphigia glabra*) extract, common grapevines (*Vitis vinifera*) seed extract (95% OPC), Ceylon cinnamon bark, zincum gluconate, D-alpha-tocopheryl acid succinate, astaxanthin (from *Haematococcus pluvialis* algae containing 5 % astaxanthin), cuprum citrate, D-biotine, L-selenomethionine. The composition comprises excipient which is Lucerne (alfalfa). The composition is in the form of a capsule, the envelope of which is prepared from hydroxypropylmethylcellulose.

All ingredients of food supplement are 100 % active substances of plant origin whithout artificial additives, binders or excipients. The composition of food supplement is administered according to the changing needs of the skin with age, it is suitable for vegan pacients.

### BRIEF DESCRIPTION OF DRAWINGS

Fig. 1. DracoBelle^{™} Nu impact on AMPK and FOXO activation
Fig. 2. Collagen levels increase with use of DracoBelle^{™} Nu
Fig. 3. Change in skin moisture and skin elasticity at baseline and after two months. The black area indicates low skin density. (High resolution ultrasound investigation)
Fig. 4. Improvement of skin parameters (in %) compared to baseline after 2 months of treatment

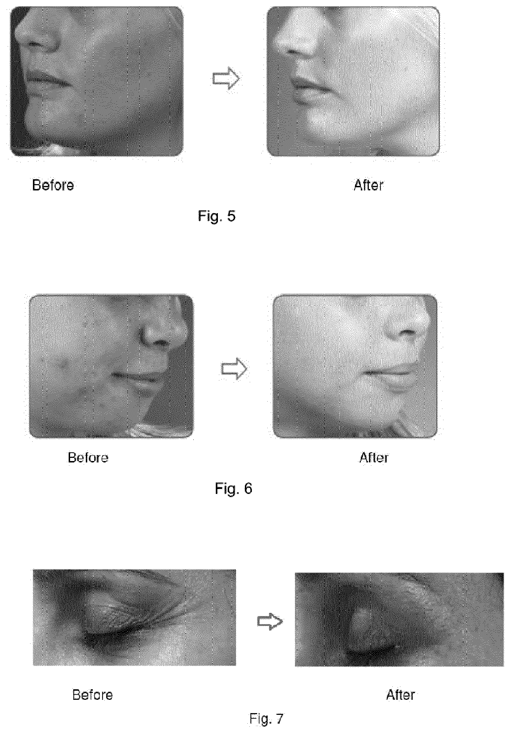

Fig. 5. Effect of the composition in case of redness of the skin
Fig. 6. Effect of the composition in the case of blemishes
Fig. 7. Effect of the composition in the presence of signs of skin aging.

### DETAILED DESCRIPTION OF INVENTION

***Moldavian dragonhead (Dracocephalum moldavica*)***,* herb extract (DracoBelle^{™}) has been invented in Swizerland laboratory "Mibelle Biochemistry". Moldavian dragonhead has a pleasant flavor and smell that is similar to lemon balm. Traditionally, Moldavian dragonhead is used as a heart protecting, antioxidant and anti-inflammatory herb. The water-soluble herbal extract from the above ground parts of organically grown Moldavian dragonhead is used. DracoBelle^{™} Nu is standardized for the active substances and has a total flavonoid glucuronide content of at least 150 µg/g. Two capsules contain 100-200 mg of Moldavian dragonhead extract to be used per day.

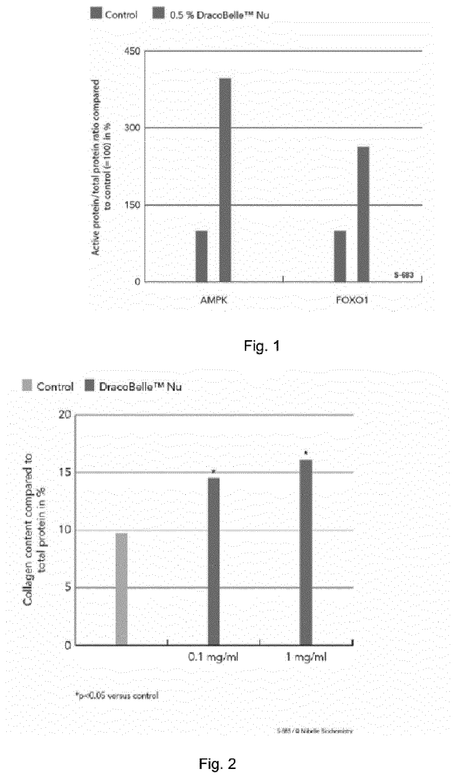

***Moldavian dragonhead extract action mechanism.*** Aging skin cells fail to clean themselves from oxidized macromolecules, defective organelles (mitochondria). Debris accumulates (polymers of lipofuscine in lyzosomes, aggregated proteins (agresomes), defective mitochondria, etc.) in the cells. The production of ATP (energy) decreases while oxidative stress increases. DracoBelle^{™} Nu inhibits the metabolic activity of skin cells, at the same time stabilizes the density of healthy skin cells and skin elasticity. The research results are based on the impact of this plant extract on the signaling pathway of AMPK kinase and the transcription factor FOX, which stabilizes skin longevity and which maintains the intracellular metabolic balance of the skin layers (synthesis / degradation, reuse of nutrients), which helps cells to get rid of defective protein proteins, and promotes skin cell regeneration [1,2].
DracoBelle^{™} Nu promotes synthesis of collagene (the effect justified by research results). An *in vitro* cell assay was performed to evaluate the effect of DracoBelle^{™} Nu on AMPK and FOXO activation. DracoBelle^{™} Nu quadrupled AMPK activity and tripled FOXO activity, as the investigation of phosphorylation levels of both proteins demonstrated (Fig. 1). DracoBelle^{™} Nu activates the ageing and longevity pathway AMPK/FOXO *in vitro* and imitate the positive effects of a low-calorie diet and healthy exercises.
In another laboratory study, nematodes were given DracoBelle^{™} Nu at concentrations of 0.1 mg/ml and 1 mg/ml or placebo. The hydroxyproline level (which reflects collagen level) in DracoBelle^{™} Nu-treated nematodes was significantly higher than that of untreated nematodes (p <0.05), thus clearly demonstrating a collagen-increasing effect.
The collagen-enhancing effect of DracoBelle^{™} Nu was concentration-dependent, resulting in a 49.8 % increase in collagen levels with 0.1 mg/ml DracoBelle^{™} Nu used and a 65.9% increase with 1 mg/ml DracoBelle ^{™} Nu used (Fig. 2).

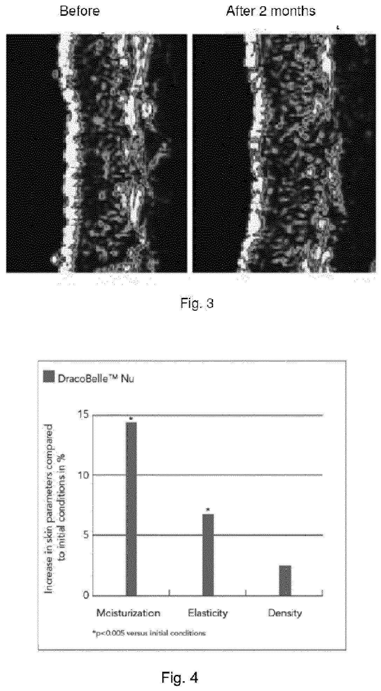

Thirty-two female volunteers (mean age = 50.8 years) with skin damaged by the sun were asked to drink a drink containing 200 mg of DracoBelle for 8 weeks. Various skin parameters in the forearm were analyzed before and after the intervention. Skin moisture and skin elasticity increased over time by 14.4 % and 6.7 %, respectively (p <0.005) (Fig. 3). Besides, skin density also improved slightly (+2.5 %). Thus, the women in the study were very satisfied with DracoBelle treatment: 94 % of the volunteers felt skin improvement, 72 % were satisfied with the performance of the product, 72 % said their skin became more hydrated (Fig. 3, 4).

***Astaxanthin.*** Astaxanthin of AstaZine^{®} is a natural astaxanthin derived from *Haematococcus pluvialis* microalgae, produced in a completely closed, 100 % glass tube, in an ecological photoreactor system. The carotenoid fraction contains 97 % pure astaxanthin and only 3 % other carotenoids. It is the world's first ecologically certified astaxanthin product (certified by Ecocert^{®}), it has as many as 7 clinical trials proving its effects on the skin and is approved as GRAS (Generally Recognized As Safe).
It is a carotenoid - an antioxidant synthesized by microalgae, which reduces skin sensitivity, redness, improves skin moisture and elasticity, reduces UV damage, mimic lines and wrinkles [3, 4]. Astaxanthin differs from other antioxidants in that it is fat-soluble and naturally penetrates into adipose tissue, such as the subcutaneous layer, where our main network of collagen and elastin fibers is located. This fat-soluble antioxidant accumulates in the subcutaneous layer and provides localized protection against free radical damage. Permanent use of fat-soluble antioxidants can drastically reduce collagen fiber ageing and prevent skin thinning and formation of wrinkles.
Astaxanthin treatment suppressed ultraviolet B (UVB)-induced inflammatory cytokine secretion in keratinocytes, and matrix metalloproteinase-1 secretion by fibroblasts cultured in UVB-irradiated keratinocyte medium. In a 16-week clinical trial with 65 healthy women, a dose of 6 mg or 12 mg of astaxanthin or placebo was administered. Wrinkle parameters and skin moisture levels worsened significantly in the placebo group after 16 weeks. Research has shown that long-term prophylactic astaxanthin supplementation may inhibit age-related skin deterioration and maintain skin conditions associated with environmentally induced damage due to its anti-inflammatory effect [22].
Astaxanthin is particularly useful for protecting skin cells from damage and maintaining the elasticity of collagen fibers.
The amount of astaxanthin is 1-3 mg in two capsules for daily use.

***Seed extract of common grapevines (Vitis vinifera)*** is standardized according to the proanthocyanidins (95 %) and therefore provide a sufficient concentration of active substances. The seed extract of common grapevines is rich in plant flavonoids proanthocyanidins (OPC). These are pigments that give taste, color and aroma to many plants and fruits. OPC effectively protects the structure of collagen in several ways. They strengthen the collagen connective tissue matrix, protect against free radical damage, and inhibit collagen damage from inflammation and infection. OPC protects against free radical damage, which is considered a major cause of the ageing process. The antioxidant effect is 50 times greater than that of vitamins C and E. The main advantage of these molecules is that they penetrate cell membranes and protect against both water- and fat-soluble free radicals. The use of combined preparations comprising *Vitis vinifera* seed extract, carotenoids, and collagen peptides isolated from microalgae increased skin thickness as measured by ultrasound and elasticity [6].
The amount of *Vitis vinifera* seed extract is 50-150 mg in two capsules for daily consumption.

**Ceylon cinnamon (*Cinnamomum zeylanicum)* bark** helps keep skin clean - studies *in vitro* revealed antimicrobial activity against acne pathogens - *Propionibacterium acnes* and *Staphylococcus epidermidis* [7]. Several pilot studies *in vitro* have shown that Ceylon cinnamon bark extract improves collagen synthesis in skin fibroblasts, i.e., may have anti-ageing effects on the skin, has antioxidant effects [8].
The amount of Ceylon cinnamon bark is 50-150 mg in two capsules for daily consumption.

***Copper*** helps to protect cells from oxidative damage, is involved in many physiological and metabolic processes, synthesis of intercellular skin matrix proteins, their stabilization and angiogenesis [9]. Copper is essential for the activation of the enzyme lysyloxidase, which is involved in the formation of cross-links between collagen and elastane (essential for healthy skin, hair, bones, blood vessels, and joints).
The copper content is 1-3 mg in two capsules for daily consumption.

***Greater burdock (Arctium lappa)*** root extract has an antioxidant-anti-inflammatory effects, inhibits the development of acne, psoriasis, eczema, has an antimicrobial activity [20, 21]. In Western medicine, burdock roots are the most valuable part of the plant for phytotherapists. Root extract regulates metabolism, lowers blood glucose, supports liver, pancreas, intestinal microflora, clean skin functions [10, 11]. Burdock has been shown to reduce certain skin disorders such as dryness or cracking, aleviating the symptoms of psoriasis and eczema.
The amount of burdock root extract is 150-300 mg in two capsules for daily consumption.

***Selenium*** is a trace element that acts as an ingredient in the antioxidant enzyme glutathione peroxidase. This enzyme works in conjunction with vitamin E and protects cell membranes from the effects of free radicals. It helps protect cells from oxidative damage, inhibits the development of skin infection *(erysipelas)* caused by *Streptococcus pyogenes,* inhibits skin ageing [12].
The selenium content is 70-150 µg in two capsules for daily consumption.

***Zinc*** helps maintain the normal condition of the skin. Studies in individuals with cutaneous acne have shown a correlation between decreased serum zinc levels in these individuals and acne [13, 14]. Several double-blinded studies have shown that zinc has a similar effect as tetracycline on superficial acne and an even better effect on deep type of acne [21]. Zinc is essential for the efficient secretion and absorption of vitamin A from the liver. Vitamin A is an extremely useful nutrient for the treatment of acne.
The amount of zinc is 15-45 mg in two capsules for daily consumption.

***Acerola cherry (Malphigia glabra) extract*** and vitamin C present in it, help maintain the normal production of collagen, which is needed for normal skin function [15]. Vitamin C enhances vitamin E activity in cells by regenerating alpha-tocopherol from oxidized derivatives. Recent cosmetology research shows that vitamin C is a powerful antioxidant that kills free radicals that damage the skin. Acerola extracts are now increasingly used in skin care products to combat cell ageing. Acerola extract is rich not only in vitamins, but also in mineral salts, which help to re-mineralize tired and sensitive skin, as well as in plant mucus and in proteins, which moisturize the skin and improve the condition of capillaries.
The amount of vitamin C is 100-300 mg in two capsules for daily consumption.

***Vitamin E*** helps protect cells from oxidative damage [16]. Vitamin E is the most important fat-soluble antioxidant found in human and animal tissues. It is found in areas of cells that are high in lipids, such as cell membranes and the brain. In these places, vitamin E is involved to stabilize and protect against oxidative damage caused by free radicals, heavy metals, and toxins in the environment.
The amount of vitamin E is 15-30 mg in two capsules for daily consumption.

***Biotin,*** which is usually classified as vitamin B, helps maintain the normal condition of the skin, hair, nails, mucous membranes, normal metabolism of macronutrients [17]. One of the first evidence that biotin is important for hair and skin was provided in a study that showed that blocking biotin uptake caused dermatitis and hair loss. Since then, it is best known that biotin is very necessary for hair, skin, and nails and that it is beneficial to take its supplements. Keratinocytes (cells in the epidermis of the skin that produce keratin, the main structural protein) use biotin, so biotin is essential for maintaining good skin condition.
The amount of biotin is 150-300 mg in two capsules for daily consumption.

***Adjuvants*** are natural, with no artificial additives, flavor enhancers or colorants. The capsule envelope is made of hydroxypropylmethylcellulose. Blue alfalfa is used as an excipient in the composition of present invention. Alfalfa leaves (*Medicago sativa*) - dried leaves are added. Alfalfa is a valuable source of nutrients right down to the roots. Alfalfa accumulates nutrients even when they are depleted by the soil due to poor agricultural practices. Alfalfa is rich in vitamins (especially vitamin K), minerals, amino acids, carotenoids, enzymes and a green pigment - chlorophyll.
The amount of blue alfalfa is 50 mg in two capsules for daily consumption.

### SKIN ELASTICITY AND MOISTURE

Collagen is a major component of the skin. It plays an important role in strengthening the skin and can also be helpful in providing skin elasticity and moisture. With age, an organism produces less collagen, therefore the skin becomes dry and wrinkles form. Numerous studies have shown that oral collagen proteins improve fibroblast growth and stimulate the production of new type I collagen in the dermis. This makes the skin smoother and softer [18]. The substances in SKIN BOOST, in particular DracoBelle^{™} Nu, astaxanthin, contribute to the production and strengthening of collagen.

### ANTIOXIDANT AND ANTI - INFLAMMATORY EFFECTS

Various skin diseases, such as acne, as well as various skin damaging factors: UV rays, chemicals in our environment, malnutrition, etc., promote the formation of free radicals that damage internal tissues and the skin, causing inflammatory processes. Gradually, the internal structure of collagen-elastin begins to break down, excess skin pigmentation is promoted, skin loses elasticity, moisture and begins to age faster. To avoid the harmful effects of inflammation, we need to suppress it.

Studies have shown that people suffering from acne-prone skin have significantly lower levels of antioxidants in the body. In addition, these people have elevated levels of inflammatory chemicals in their blood. Patients suffering from acne face severe systemic inflammation and intense oxidative stress that their antioxidants cannot encounter with. Additional use of antioxidant substances would reduce the concentration of free radicals, protect the skin from further damage and reduce the inflammatory process.

The antioxidants in the composition, which help protect the skin from free radicals and reduce inflammation, are: DracoBelle^{™} Nu herb extract, selenium, astaxanthin, natural vitamin E, zinc, copper, common grapevines seed extract, natural Vitamin C, greater burdock root extract.

### PROTECTION AGAINST ACNE

Zinc is very important in the regulation of sebaceous gland activity. Excessive fat secretion is a separate factor in many skin problems. Acne is caused by excessive secretion of fat, which clogs the hair follicles, accumulates bacteria, causing infection and inflammation. Many cases of dry skin involve decreased activity of the sebaceous glands. Zinc regulates the activity of the sebaceous glands, so if the glands produce too much fat, adequate intake of zinc reduces the amount of fat secreted. On the contrary, if the amount of fat excreted is insufficient, consuming the right amount of zinc will help improve fat production. One double-blind, placebo-controlled study revealed that as many as 58 % of participants in the group who took zinc supplements had a drastic reduction in acne formation [20].

### LIVER HEALTH

Burdock roots have been used for centuries to improve skin health. Their effects on skin health are thought to be related to the positive effects on the liver. The relevance of the liver to skin health is undeniable. Burdock roots contain many bitter compounds that promote bile production and excretion. It is one of the main means of transporting fat-soluble waste from the liver. This increases the efficiency of liver function and at the same time contributes to the promotion of skin health. Because bile is a natural emulsifier, increased bile flow will increase the uptake and utilization of dietary fatty acids. This will help to improve the texture and appearance of the skin. Burdock is observed to have a mild anti-inflammatory effect.

### COMPOSITION AND METHOD OF PREPARATION THEREOF

| Nutrient | 2 capsules contain: | RNV* % |
|---|---|---|
| DracoBelle^{™} Nu | 100 - 200 mg | - |
| Astaxanthin of plant origin from algae (AstaZine^{®}, containing astaxanthin 5 %) | 1-3 mg | - |
| Common grapevines (*Vitis vinifera*) seed extract 95 % OPC | 50-150 mg | - |
| Ceylon cinnamon (*Cinnamomum zeylanicum*) bark | 50-150 mg | - |
| Copper | 1-3 mg | 200 |
| Greater burdock root extract (4:1) | 150-300mg | - |
| Zinc | 15-45 mg | 300 |
| Selenium | 70-150 µg | 200 |
| Vitamin C (from acerola cherry extract) (25 % of vitamin C) | 100-300 mg | 200 |
| Vitamin E | 15-30 mg α-TE (30 IU) | 250 |
| Biotin | 150-300 µg | 400 |

| | | |
|---|---|---|
| * (RNV) -Reference nutritional value | | |

### A METHOD FOR THE PREPARATION OF COMPOSITION

The composition provided above is prepared by mixing all the ingredients by methods known in the art. All ingredients are prepared in powder form.

First, the extracts are combined and mixed: 200 mg of Moldavian dragonhead extract, 200 mg of greater burdock root extract, 160 mg of acerola cherry extract, 100 mg of common grapevines seed extract. The remaining crushed ingredients are then added: 2 mg of astaxanthin, 100 mg of Ceylon cinnamon bark, 30 mg of zinc gluconate, 20 mg D-alpha-tocopheryl acid succinate, 2 mg of copper citrate, 200 µg of D-biotin, 110 µg of L-selenomethionine. At the end, 50 mg of blue alfalfa is added as an excipient. The amounts are for two capsules, which are administered daily.

The mixture is mixed well and filled into capsules. The capsule envelope consists of hydroxypropylmethylcellulose.

The composition is intended for:
- skin nourishment, improvement of elasticity and protection against oxidative damage,
- inhibition of ongoing ageing processes, inhibition of wrinkle formation,
- improvement of the condition of the skin in case of: skin inflammation, acne, redness of the skin, red blemishes, scars, etc.

### EFFECTS OF THE COMPOSITION

Following a one-month washout period, subjects received two anti-ageing skin care formula tablets daily for 16 weeks (total daily dose: marine complex 210 mg, vitamin C 54 mg, zinc 4 mg) or placebo. Subjects were evaluated at weeks 6, 12, and 16 for clinical evaluation (0-10 VAS), bioinstrumentation, digital photography, and self-evaluation. Finally, it was concluded that women who have skin damaged by photoageing have significantly improved skin condition after treatment with dietary supplements that regulate skin ageing.

### PHOTOGRAPHIC EXAMPLES

Patients have been studied comparing skin condition before and after the treatment. The patients were administered the dietary supplement containing the following ingredients per capsule: 100 mg Moldavian dragonhead (*Dracocephalum moldavica*) extract (1:18), 150 mg of greater burdock (*Arctium* lappa) root extract (4: 1), 150 mg of acerola cherry (*Malphigia glabra*) extract, 75 mg of common grapevines (*Vitis vinifera*) seeds extract (95 % OPC*), 75 mg of Ceylon cinnamon (*Cinnamomum zeylanicum*) bark, 20 mg of zinc gluconate, 10 g of D-alpha-tocopheryl acid succinate, 1 mg of astaxanthin (from *Haematococcus pluvialis* algae) (containing astaxanthin 5 %), 1 mg of copper citrate, 100 µg D-biotin, 70 µg L-selenomethionine; capsule envelope: hydroxypropylmethylcellulose.

### Fig. 5

The patient (25-year-old woman) with reddening of the skin was given the food supplement twice a day for 3 weeks. Significant improvement in skin condition is observed after the treatment period.

### Fig. 6

The patient (21-year-old woman), with blemishes on the face, was given the said food supplement twice a day for 1 month. After consuming the supplement, the face became smoother, without blemishes, brighter.

### Fig. 7

The patient (54-year-old woman), with the presence of signs of skin ageing, was given the said food supplement twice a day for 1 month. Significant improvement in skin condition is observed after the administration of the food supplement.

### Skin hydration

When using the food supplement, patients feel significantly softer, more elastic, and more pleasant skin.

The composition of the invention is effective in improving the condition of the skin in the presence of skin inflammation, acne, reddening of the skin, red blemishes, scars and the like. The said composition is effective in nourishing the skin, improving skin elasticity and protecting against oxidative damage. The said composition inhibits the ongoing aging processes, inhibits the formation of wrinkles.

## Claims

1. Skin care composition for oral administration, **characterized in that** the composition comprises at least:
(a) extract of Moldavian dragonhead (*Dracocephalum moldavica*);
(b) extract of greater burdock (*Arctium lappa*) roots;
(c) extract of grapevines (*Vitis vinifera*) seeds;
(d) cinnamon;
where the composition essentially does not contain any artificial additives, artificial binders or excipients;
where the composition further comprises agents protecting cells from oxidative stress; agents maintaining normal skin condition; the agent maintaining normal collagen formation.

2. Skin care composition according to claim 1, **characterized in that** the agents protecting skin cells from the oxidative stress are copper, selenium, and vitamin E.

3. Skin care composition according to claim 1, **characterized in that** the agents maintaining the normal skin condition are zinc and biotin.

4. Skin care composition according to claim 1, **characterized in that** the agent maintaining normal collagen formation is vitamin C.

5. Skin care composition according to any one of previous claims, **characterized in that** the composition is prepared in the form of capsule.

6. Skin care composition according to any one of previous claims, **characterized in that** the envelope of capsule is prepared of hydroxypropylmethylcellulose.

7. Skin care composition according to any one of previous claims, **characterized in that** the capsule comprises excipients.

8. Skin care composition according to any one of previous claims, **characterized in that** the excipient of the capsule is lucerne.

9. Skin care composition according to any one of previous claims **characterized in that** the composition is for the formation of collagen in the skin.

10. Skin care composition according to any one of previous claims for use in inhibiting the processes of skin aging and wrinkles formation.

11. Skin care composition according to any one of previous claims for use in nourishing the skin, improving elasticity, and protecting against oxidative damage.

12. Skin care composition according to any one of previous claims for use in improving the condition of the skin in the presence of: skin inflammation, acne, redness of the skin, red blemishes and scars.
